# EUROPEAN PATENT SPECIFICATION

(11) **EP 1 519 771 B1**
(45) Date of publication and mention of the grant of the patent: **19.05.2010**
(21) Application number: 03727244.0
(22) Date of filing: 27.05.2003
(51) Int. Cl.: A61N 5/06, A61B 18/18

(54) **THERAPEUTIC TREATMENT DEVICE WITH INCOHERENT LIGHT SOURCES**
THERAPEUTISCHE BEHANDLUNGS- VORRICHTUNG MIT INKOHÄRENTEN LICHTQUELLEN
DISPOSITIF DE TRAITEMENT THERAPEUTIQUE COMPRENANT DES SOURCES DE LUMIERE INCOHERENTE

(30) Priority: 08.07.2002 DK 200201075
(43) Date of publication of application: 06.04.2005
(73) Proprietor: Cyden Ltd., Swansea SA1 8PH (GB)
(72) Inventor: SIMONSEN, Jan, Henning, DK-7600 Struer (DK)
(74) Representative: Austin, Hedley William
(86) International application number: PCT/DK2003/000349
(87) International publication number: WO 2004/004831

(56) References cited:
- EP-A- 1 038 505
- EP-A- 1 078 604
- EP-A- 1 238 683
- US-A- 5 879 346
- DATABASE WPI Section PQ, Week 200015 Derwent Publications Ltd., London, GB; Class P34, AN 2000-170079 XP002251569 & RU 2 122 449 C (NIZHEGOROD MED ACAD), 27 November 1998 (1998-11-27)
- DATABASE WPI Section PQ, Week 200032 Derwent Publications Ltd., London, GB; Class P34, AN 2000-374109 XP002251570 & RU 2 134 601 C (GRABOVSHCHINER A YA), 20 August 1999 (1999-08-20)

## Description

The present invention relates to a therapeutic treatment device for non-invasive treatment of skin-disorders such as discolouring, acne, wrinkles, blood vessels, cellulite and stretch marks. Furthermore the device can be used to remove unwanted hair, softening of scar tissue and other skin-disorders.

### Background of the invention

It is known in the prior art to use magnetic radiation in medical application for therapeutic uses such as treatment of skin disorders. For example, US patent No. 4,298,005 to Mutzhas describes a continuous ultraviolet lamp with cosmetic, photo biological, and photochemical applications. A treatment based on using the UV portion of the spectrum and its photochemical interaction with the skin is described. The power delivered to the skin using Mutzhas' lamp is described as 150 W/m², which does not have a significant effect on skin temperature.

In addition to prior art treatment involving UV light, lasers have been used for dermatological procedures, including Argon lasers, CO₂ lasers, Nd(Yag) lasers, copper vapour lasers, ruby lasers and dye lasers. For example, US patent No. 4,829,262 to Furumoto, describes a method of constricting a dye laser used in dermatology applications. Two skin conditions which may be treated by laser radiation are external skin irregularities such as local differences in the pigmentation or structure of the skin, and vascular disorders lying deeper under the skin which cause a variety of skin abnormalities including port wine stains, telangiectasias, leg veins and cherry and spider angiomas. Laser treatment of these skin disorders generally includes localised heating of the treatment area by absorption of laser radiation. Heating the skin changes or corrects the skin disorder and causes the full or partial disappearance of the skin abnormality.

Certain external disorders such as pigmented lesions can also be treated by heating the skin very fast to a high enough temperature in order to evaporate parts of the skin. Deeper-lying vascular disorders are more typically treated by heating the blood to a high enough temperature to cause it to coagulate. The disorder will then eventually disappear. To control the treatment depth a pulsed radiation source is often used. The depth the heat penetrates in the blood vessel is controlled by controlling the pulse wave band width of the radiation source. The absorption and scattering coefficients of the skin also affect the heat penetration. These coefficients are a function of the constituents of skin and the wavelength of the radiation. Specifically, the absorption coefficient of light in the epidermis and dermis tends to be a slowly varying, monotonically decreasing function of wavelength. Thus, the wavelength of the light should be conditioned according to the vessel size being treated.

The effectiveness of lasers for applications such as tattoo removal and removal of birth and age marks is diminished because lasers are monochromatic. A laser of a given wavelength may be effectively used to treat a first type of skin pigmentation disorder, but, if the specific wavelength of the laser is not absorbed efficiently by skin having a second type of disorder, it will be ineffective for the second type of skin disorder. Also, lasers are usually complicated, expensive to manufacture, large in comparison to the amount of power delivered, unreliable and difficult to maintain.

The wavelength of the light also affects vascular disorder treatment because blood content in the vicinity of the vascular disorders varies, and blood content affects the absorption coefficient of the treatment area. Oxyhemoglobin is the main chromophore which controls the optical properties of blood and has strong absorption bands in the visible region. More particularly, the strongest absorption peak of oxyhemoglobin occurs at 418 nm and has a band-width of 60 nm. Two additional absorption peaks with lower absorption coefficients occur at 542 and 577 nm. The total band-width of these two peaks is in the order of 100 nm. Additionally, light in the wavelength range of 500 to 550 nm is desirable for the treatment of blood vessel disorders of the skin since it is absorbed by the blood and penetrates through the skin. Longer wavelengths up to 1000 nm are also effective since they can penetrate deeper into the skin, heating the blood vessel by thermal conductivity. Also, longer wavelengths are effective for treatment of larger diameter vessels because the lower absorption coefficient is compensated for by the longer path of light in the vessel.

Especially when treating vascular disorders in the vicinity of the skin surface, it was found during development of the present device that treatment in the red spectrum turned the haemoglobin instantly into oxyhemoglobin. Oxyhemoglobin is easily detectable as a black colouring. As this takes place in the entire area treated and as it spreads out with the bloodstream and furthermore lasts for a period of time, this effect is undesirable.

Document EP-A-1 078 604 discloses a device including an incoherent light source such a flashlamp and a reflector.

### Object of the invention

Accordingly, a wide band electromagnetic radiation source that covers the near UV and the visible portion of the spectrum would be desirable for treatment of external skin and vascular disorders. The overall range of wavelengths of the light source should be sufficient to optimise treatment for any of a number of applications. Such a therapeutic electromagnetic radiation device should also be capable of providing an optimal wavelength range within the overall range for the specific disorder being treated. The intensity of the light should be sufficient to cause the required internal and/or external thermal effect by raising the temperature in or around the treatment area to the required temperature, but without causing pain or skin problems. Also, the pulse-width should be variable over a wide enough range so as to achieve the optimal penetration depth for each application.

Therefore, it is desirable to provide a therapeutic treatment device comprising one or more light sources having a wide range of wavelengths, which can be selected according to the required skin treatment, with a controlled pulse band width and a high enough energy density for application to and in the affected area.

### Summary of the invention

Such a therapeutic device is defined in claim 1.

Also for stimulating the skin's collagen content and production it is necessary to direct the light energy to these specific areas at specific wave lengths in the skin, such that the collagen production will be stimulated. Increased collagen production helps the skin to remain flexible and smoothen out wrinkles or retard the formation of wrinkles. By combining different types of light sources, it is possible to provide variable intensities at different specific wave lengths and thereby optimise the treatment.

Furthermore, tests have indicated that the blood's ability to produce collagen can be stimulated by introducing energy into the skin tissue. As the collagen helps in achieving a more supple tissue, which improves healing of scares as well as prevents acne and other skin disorders this is a desirable feature. By stressing the capillary blood vessels almost to the damage level during a cycle of treatments, the collagen production can be greatly improved. Therefore, treatment with a device comprising suitable light source means and appropriate filter means can improve skin healing. The treatment should be carried out both before and after scar formation.

In a further advantageous embodiment the device is arranged such that each light source can be controlled independently of the other light sources. It is hereby possible to design a sequence of different light flashes such that during the treatment, the sequence of flashes is designed in order to optimise the effect of the delivered energy to the skin.

One of the light sources is designed to emit a series of light pulses. For most treatments the amount of energy delivered to the skin is decisive on the successful treatment. Especially when removing unwanted hair or treating other disorders, it is important to deliver the maximum amount of energy in a very short time span, such that the energy intensity is maximised. On the other hand, it is also important to limit the amount of delivered energy such that unwanted side effects in the shape of pain burns, discolouring arising from the light treatment etc. can be avoided. In this connection, it has been found that by rapidly flashing one device or a series of many devices rapidly one after the other, the energy intensity in the zone which has to be treated can be greatly increased without the risk of the side effects. It hereby becomes possible to cany out an effective treatment without the risk of side effects.

In a still further advantageous embodiment a filter can be arranged such that the emitted light will travel through the filter. In this way it is possible to select the optimum band of wavelengths which will have the most effective treatment according to the disorder which is to be treated. For example it is known that water absorbs energy at the wavelength 1015 nm. In order to avoid overheating in the skin it is desirable to avoid pulsing too much light energy at this wave length into the skin, whereby over heating due to water's absorption of the energy can be avoided, and therefore a more efficient treatment of the disorder can be achieved instead of spending the energy on heating the skin's content of water. A similar relationship is applicable for treating vascular disorders such as blood vessels immediately under the skin or rosacea which is an acne-like disease, often in central parts of the face. For treatment of these kinds of disorders it is interesting to be able to use specific wavelengths for treating the bloods vessels under the skin, and for stimulating the collagen production.

In a still further advantageous embodiment different filters or no filter at all can be arranged with respect to every distinct light source. Hereby it becomes possible to design the light treatment for a specific purpose.

As the light influences the skin's condition, especially in the outermost layers of the skin, the effective characteristics of the skin also changes. This in turn has influence on the effectiveness of the light treatment in that the skin's ability to absorb the transmitted energy varies according to the wavelength of the energy source and the composition of the tissue. Some of these changes in the skin characteristics are almost instantaneous.

In an embodiment of the invention a filter comprising different sections/areas is used. Each area/section filters light in a specific wavelength range and is moved across the light source during the light emitting sequence. By moving the appropriate filter at the right velocity across the light emitter, it is possible to transmit a high and constant level of energy into the tissue, even when the tissue's characteristics concerning absorption ability and reflectivity changes. Depending on the skin type, type of treatment and selected light emitting means/sequence, the filter can be designed accordingly as the changes in the skin tissue are known.

By combining the above mentioned features with the feature that each light source can be controlled independently with rested to intensity, pulse length, wavelength time delay for executing each light source in relation to another or time lapsed between each flash in a multi-flash device, the light treatment can be completely controlled and designed according to the optimum treatment.

A special problem arises when treating darker skin. These skin types typically have a high content of melanin, the colouring ingredient in skin. Also, darker skin types usually have darker hair. For treating these skin and hair types, more energy needs to be delivered to the skin. Hereby, the danger of causing burns, pain or other forms of irritation arises. These skin types can advantageously be preheated up to 50-60°C or even 80°C before the light treatment. Temperature means, for example in the shape of an infrared thermometer, may be arranged and, optionally, connected to a control circuitry in the device for controlling the skin and/or tissue temperature in the treatment area.

Tests have indicated that it is the difference in temperature within the skin which causes damage to the tissue. Therefore, by preheating the area around the treatment area, the temperature difference as well as the temperature gradient between not heated tissue and the treatment area can be minimized while at the same time it is possible to deliver enough energy into the desired skin layer or hair follicle for successful treatment.

In another embodiment between four and ten light sources are arranged in a reflector unit and lens means is arranged in front of the reflector. This embodiment is especially advantageous as first of all the light sources are presented as one single unit, namely the reflector closed off by a lens element, but also by the necessary light sources in the same unit. A complete treatment can be facilitated by this finished unit. The lens element can be designed either alone or in cooperation with the reflector to target the emitted light at one well defined target area to be treated.

In practice, the light sources are selected among flash bulbs and electronic flash tubes and maybe others.

The energy delivered to the skin is desired to be around 2 joules per cm² per flash. This energy density is high enough to have an effect on the skin disorders described above, but also it is not enough to cause damage to normal skin disorders or skin types. By combining different filters and selecting different intensities it is possible to take into consideration the type of skin to be treated i.e. fair skin should have lower intensity than darker skin. The treatment device can be applied in a number of applications for either therapeutic treatment or cosmetic treatment. By selecting and/or combining different light sources with different wavelengths and intensities, designing these in a specific sequence and pulse length it is possible to design light treatment for the skin disorders mentioned above. For removal of hair it is possible to first pulse or heat up the hair follicle, overlay the initial heating with a stronger light pulse whereby the hair sac will be destroyed. In this fashion it is possible to combine and after the sequence between flash bulb devices and electronic flashes completely freely according to the most optimum treatment.

The light sources are arranged in one replaceable/disposable unit. This is an especially advantageous feature with the present invention in that the device itself includes the electrical energy source and the trigger mechanism, and can be used for multiple treatments for multiple disorders by simply selecting the appropriate light sources designed for that specific treatment. The device can be designed as a mobile handheld device whereby treatment can be carried out anywhere.

In a former advantageous embodiment of the light emitting device, the constellation of different light source in a device constitutes a complete specific treatment. Hereby it is possible for the uses to acquire a number of disposable units for repeated treatment or treatment of larger areas, and furthermore treatment for the specific skin-disorder. As explained above, different skin-disorders are due to different causes in the skin or fat tissue which are all susceptible to treatment by different wavelengths. By designing the light emitting devices such that they will emit the most optimum wavelengths as well as controlling the sequence of light emissions and the intensity of the light emitted, it will be possible in one disposable unit to design a specific treatment.

Furthermore, by having knowledge about the energy levels required in order to treat fair skin in comparison to dark skin it is possible to design the disposable light emitting devices such that the user will be able to acquire complete treatment, especially tailored for their type of skin.

The light sources are arranged in a replaceable/disposable reflector unit. The light emitting device is hereby equivalent to a regular light bulb. Once the light emitting device has been activated and spent it can be disposed of very easily by replacing the entire bulb comprising one or more light sources and installing a new light emitting device either for treating the same kind of skin-disorder or treating a different kind of skin-disorder as described above.

The light emitting device comprises means for controlling the sequence and/or the pulse length, and/or intensity of each light emitting source. By incorporating control means for example in the form of a microchip and microswitches it is possible, as explained above, to ignite different light sources at different times whereby a multitude of different treatments can be carried out according to the programming of the control means.

Tests have shown that disposable flash lights emit light of an intensity which provides enough energy to have en effect on the skin. Likewise has testing of electronic flash devices comparable to those used in disposable cameras proved to be effective enough in order to be able to provide a multiple flash device for use in the present invention.

Tests have shown that by designing the light emission by varying the intensity, the pulse length and the wavelength, it is also possible to have an effect on the treatment of psoriasis as well as birthmarks.

In connection with plastic surgery and especially for the healing of scars after surgical invasion, it has shown that the light treatment which stimulated the collagen production improves the healing process and minimizes scar tissue, whereby the traces of either plastic surgery or regular surgery will be minimized. The treatment can also be carried out before the surgery in order to prepare the skin in the effected area to be more flexible and stimulative and with a high collagen content.

In one example of a treatment according to the invention, visible blood vains present in the lower leg area were treated. A device according to the invention was used. First, a series of "warming up" shots by an electronic flash device were directed against the area to be treated. With the prototype it is possible to treat an area of approximately up to 1600 mm² in one cycle. After the initial heating up of the area, an energy intense flash, i.e. up to 2 J/cm² was directed at the treatment area.

The process which is accelerated or initiated in the skin is that in the warming up phase the blood is heated and a "blue response" phenomenon appears. This is due to the haemoglobin comprising oxygen is deoxidised whereby the blue colour appears in the area. During exposure to the actual treatment flash dose the haemoglobin in the blood in the treatment area looses an oxygen molecule and hooks up with a Fe-molecule, whereby so-called met-haemoglobin is created. This is detected as a black colouring in the treatment area.

To the patient the flash treatment is conceived as the blood veins in the vicinity of the skin surface will be destroyed. The blood will coagulate and created what looks like a severe bruise (black and blue). As the veins are destroyed, the normal blood circulation in the area will transport the met-haemoglobin away from the area, which after a period of time will regain its normal skin tone.

## Claims

1. Therapeutic treatment device for non-invasive treatment of skin disorders or removal of unwanted hair, said device comprising one or more light sources for emitting incoherent light arranged in a reflector unit, the device further including
(i) an energy source for providing an electrical current to said light source;
(ii) a trigger mechanism ; and
(iii) means for controlling the intensity and/or pulse length and/or sequence of each said light source, at least one said light source being arranged to emit a series of light pulses, **characterized in that** the reflector unit, including the light sources one or more is replaceable after activation thereof and disposable after such replacement.

2. A device according to claim 1, which includes four to ten said light sources arranged in said reflector unit and lens means are arranged in front of a reflector such that the reflector is closed off by the lens means forming said reflector unit.

3. A device according to claim 1 or 2, **characterised in that** a filter is arranged in the device such that emitted light will travel through the filter for said treatment.

4. A device according to any of claims 1 to 3, **characterised in that** each said individual light source comprises an electronic flash tube or flash bulb.

## Patentansprüche

1. Therapeutische Behandlungsvorrichtung zur nicht-invasiven Behandlung von Hautstörungen oder zur Beseitigung von unerwünschtem Haar, wobei die Vorrichtung eine oder mehrere Lichtquellen zur Emission inkohärenten Lichtes umfasst, die in einer Reflektoreinheit angeordnet sind, wobei die Vorrichtung ferner enthält:
(i) eine Energiequelle, um die Lichtquelle mit einem elektrischen Strom zu versorgen;
(ii) einen Triggermechanismus; und
(iii) ein Mittel zur Steuerung der Intensität und/oder der Pulslänge und/oder der Folge einer jeden Lichtquelle, von mindestens einer Lichtquelle, die so angeordnet ist, um eine Serie von Lichtimpulsen zu emittieren,
**dadurch gekennzeichnet, dass** die Reflektoreinheit, welche die mehrere Lichtquellen enthält, nach Aktivierung derselben austauschbar ist und nach einem solchen Austausch wegwerfbar ist.

2. Vorrichtung nach Anspruch 1, die vier bis zehn der Lichtquellen enthält, die in der Reflektoreinheit angeordnet sind, und ein Linsenmittel sind vor einem Reflektor so angeordnet, dass der Reflektor durch das Linsenmittel abgeschlossen wird, das die Reflektoreinheit ausbildet.

3. Vorrichtung nach Anspruch 1 oder 2, **dadurch gekennzeichnet, dass** ein Filter in der Vorrichtung so angeordnet ist, dass das emittierte Licht durch den Filter für die Behandlung hindurchgehen wird.

4. Vorrichtung nach einem der Ansprüche 1 oder 3, **dadurch gekennzeichnet, dass** jede einzelne Lichtquelle eine (elektronische) Blitzröhre oder eine Blitzbirne umfasst.

## Revendications

1. Dispositif de traitement thérapeutique, pour le traitement non invasif de troubles cutanés, ou pour l'élimination de poils non souhaités, ledit dispositif comprenant une ou plusieurs sources de lumière destinées à émettre une lumière incohérente disposées à l'intérieur d'une unité de réflecteur, le dispositif comprenant en outre :
(i) une source d'énergie destinée à fournir un courant électrique à ladite source de lumière;
(ii) un mécanisme de déclenchement ; et
(iii) des moyens de contrôle de l'intensité et/ou de la longueur d'impulsion et/ou de la séquence de chacune desdites sources de lumière, au moins une source de lumière étant configurée pour émettre une série d'impulsions lumineuses,
**caractérisé en ce que** l'unité de réflecteur, comprenant la ou plusieurs sources de lumière, peut être remplacée après son activation et peut être jetée après un tel remplacement.

2. Dispositif selon la revendication 1, qui comprend quatre à dix desdites sources de lumière disposées dans ladite unité de réflecteur et des moyens de lentilles qui sont disposés devant un réflecteur de telle manière que le réflecteur est bloqué par les moyens de lentille formant ladite unité de réflecteur.

3. Dispositif selon la revendication 1 ou 2, **caractérisé en ce qu'**un filtre est disposé dans le dispositif de telle manière que la lumière émise traversa le filtre pour ledit traitement.

4. Dispositif selon l'une quelconque des revendications 1 à 3, **caractérisé en ce que** chaque source de lumière individuelle comprend un tube électronique à éclairs ou une lampe électronique à éclairs.
